(19)

**Europäisches Patentamt**

**European Patent Office**

**Office européen des brevets**

(11) **EP 3 422 222 B1**

(12) **EUROPEAN PATENT SPECIFICATION**

(45) Date of publication and mention
of the grant of the patent:
**10.04.2024  Bulletin 2024/15**

(21) Application number: **17178771.6**

(22) Date of filing: **29.06.2017**

(51) International Patent Classification (IPC):
**G16H 40/63** *(2018.01)*    **G16H 50/20** *(2018.01)*
**G16H 50/30** *(2018.01)*    **G16H 50/70** *(2018.01)*

(52) Cooperative Patent Classification (CPC):
**G16H 50/20; G16H 40/63; G16H 50/30;**
**G16H 50/70;** G16H 40/40

(54) **METHOD AND STATE MACHINE SYSTEM FOR DETECTING AN OPERATION STATUS FOR A SENSOR**

VERFAHREN UND ZUSTANDSMASCHINENSYSTEM ZUR DETEKTION EINES BETRIEBSSTATUS FÜR EINEN SENSOR

PROCÉDÉ ET SYSTÈME DE MACHINE D'ÉTAT POUR DÉTECTER UN ÉTAT DE FONCTIONNEMENT D'UN CAPTEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**02.01.2019   Bulletin 2019/01**

(73) Proprietors:
• **Roche Diabetes Care GmbH**
  **68305 Mannheim (DE)**
  Designated Contracting States:
  **AT BE BG DE DK EE FI FR IT LT LU LV MT NL PT SE SI**
• **F. Hoffmann-La Roche AG**
  **4070 Basel (CH)**
  Designated Contracting States:
  **AL CH CY CZ ES GB GR HR HU IE IS LI MC MK NO PL RO RS SK SM TR**

(72) Inventors:
• **Rueckert, Frank**
  **68305 Mannheim (DE)**
• **Nuernberg, Frank-Thomas**
  **68163 Mannheim (DE)**
• **Weilbach, Juliane**
  **68199 Mannheim (DE)**

(74) Representative: **Bittner, Thomas L.**
**Boehmert & Boehmert**
**Anwaltspartnerschaft mbB**
**Pettenkoferstrasse 22**
**80336 München (DE)**

(56) References cited:
**US-A1- 2010 323 431     US-A1- 2014 182 350**
**US-A1- 2015 164 386**

**Description**

The present disclosure refers to a method and a state machine system for determining an operation status for a sensor.

Background

Document US 2014 / 0182350 A1 discloses a method for determining the end of life of a CGM (continuous glucose monitoring) sensor including evaluating a plurality of risk factors using an end of life function to determine an end of life status of the sensor and providing an output related to the end of life status of the sensor. The plurality of risk factors are selected from a list including a number of days the sensor has been in use, whether there has been a decrease in signal sensitivity, whether there is a predetermined noise pattern, whether there is a predetermined oxygen concentration pattern, and an error between reference BG (blood glucose) values and EGV sensor values.

Document EP 2 335 584 A2 relates to a method for self-diagnostic test and setting a suspended mode of operation of the continuous analyte sensor in response to a result of the self-diagnostic test.

In document US 2015 / 164386 A1, electrochemical impedance spectroscopy (EIS) is used in conjunction with continuous glucose monitors and continuous glucose monitoring (CGM) to enable in-vivo sensor calibration, gross (sensor) failure analysis, and intelligent sensor diagnostics and fault detection. An equivalent circuit model is defined, and circuit elements are used to characterize sensor behavior. This document represents the closest prior art.

Document US 2010 / 323431 A1 discloses a control circuit and method for controlling a bi-stable display having bi-stable segments each capable of transitioning between an on state and an off state via application of a voltage. The voltage is provided to a display driver from a charge pump, and supplied to individual ones of the bi-stable segments via outputs from the display driver in accordance with display instructions provided by a system controller. Both a bi-stable segment voltage level of at least one of the outputs of the display driver and a charge pump voltage level of the voltage are detected and compared to a valid bi-stable segment voltage level and a valid charge pump voltage level, respectively. A malfunction signal may be provided to the system controller if either of the detected voltage levels is not valid.

Summary

It is an object of the present disclosure to provide a state machine system and a method for detecting an operation status for a sensor which will allow to predict potential operation status problem more safely.

For solving the problem a method for detecting an operation status for a sensor according to the independent claim 1 is proposed. Further, a state machine system for performing a method for detecting an operation status for a sensor according to the independent claim 12 is provided. Alternative embodiments are the subject of dependent claims.

According to an aspect, a method for detecting an operation status for a sensor is provided. In a state machine, the method comprises: receiving continuous monitoring data related to an operation of a sensor, providing a trained learning algorithm for detecting an operation status for the sensor which signifies a sensor function, wherein the learning algorithm is trained according to a training data set comprising historical data, detecting an operation status for the sensor by analyzing the continuous monitoring data with the trained learning algorithm, and providing output data indicating the detected operation status for the sensor.

According to further aspect, a state machine system is provided. The state machine system is having one or more processors configured for data processing and for performing a method for detecting an operation status for a sensor, the method comprising: receiving continuous monitoring data related to an operation of a sensor, providing a trained learning algorithm for detecting an operation status for the sensor which signifies a sensor function, wherein the learning algorithm is trained according to a training data set comprising historical data, detecting an operation status for the sensor by analyzing the continuous monitoring data with the trained learning algorithm, and providing output data indicating the detected operation status for the sensor.

According to the technologies proposed, a process of machine learning is applied for detecting operation status of the sensor. Thereby, a predictive method is implemented for determining the operation status of the sensor by using a trained learning algorithm trained according to a training data set and applied for analyzing continuous monitoring data related to the operation of the sensor.

For example, abnormalities and / or malfunctions with regard to the operation of the sensor may be predicted, thereby avoiding potential problems in the operation of the sensor.

The learning algorithm is trained according to the training data set comprising historical data. The term "historical data" as used in the present application refers to data collected, detected and / or measured prior to the process of determining the operation status. The historical data may have been detected or collected prior to starting collection of the continuous monitoring data received for operation status detection.

The training data set may be collected, detected and / or measured by the same sensor and / or by some different sensor. The sensor different from the sensor for which the operation status is detected may be of the same sensor type.

The training data set may comprise training data indicative of a sensor status to be detected or predicted. For example, the training data set may be indicative of one or more of the following: a manufacturing fault status, malfunction status, a glycemic indicating status, and an anamnestic indicating status.

The detecting may comprise at least one of detecting a manufacturing fault status for the sensor indicative of a fault in a process for manufacturing the sensor, detecting a malfunction status for the sensor indicative of a malfunction of the sensor, detecting an anomaly status for the sensor indicative of an anomaly in operation of the sensor, detecting a glycemic indicating status for the sensor indicative of a glycemic index for a patient for whom the continuous monitoring data are provided; and detecting an anamnestic indicating status for the sensor indicative of an anamnestic patient status for the patient for whom the continuous monitoring data are provided. The detecting of the manufacturing fault status for the sensor may be performed after manufacturing the sensor. Alternatively or in addition, the detecting of the manufacturing fault status may be applied to an intermediate sensor product (not finalized sensor) while the manufacturing process is still running. Similarly, the detecting of the malfunction status for the sensor may be part of or related to the manufacturing process. Alternatively, by the technology proposed, a malfunction status for the sensor may be predicted after the manufacturing process has been finalized, for example in case of applying the sensor for measurement. The detecting of the anomaly status for the sensor may be done in a measurement process, for example in real time while detection of measurement signals by the sensor is going on. Similarly one of the detecting of the glycemic indicating status and the detecting of the anamnestic indicating status may be performed while a measurement process is running. Alternatively, such detecting may be applied after a measurement process has been finished.

A glycemic index may be determined for the patient, for example, in response to detecting the glycemic indicating status for the sensor. The glycemic index is a number associated with a particular type of food that indicates the foods effect on a person's blood glucose (also called blood sugar) level. A value of one hundred may represent the standard, an equivalent amount of pure glucose. In addition or as an alternative, other glycemic parameters may be determined, such parameters including rate-of-change of blood glucose level, acceleration, event patterns due to, for example, movement of the patient, meal, mechanical stress on the sensor with regard to the anamnestic indicating status for the sensor. With regard to the anamnestic indicating status, potentially anamnestic data may be determined such as hba1c or demographic data like age and / or sex of the patient.

Providing the trained learning algorithm may comprise providing at least one learning algorithm selected from the following group, K-nearest neighbor, support vector machines, naive bayes, decision trees such as random forest, logistic regression such as multinominal logistic regression, neuronal network, decision trees, and bay's network. Of preferred interest may be one of naive bayes, random forest, and multinominal logistic regression. In a preferred embodiment the random forest algorithm may be applied for which correlation and interactions between parameters analyzed or automatically incorporated.

In this embodiment a method comprises the training of the learning algorithm according to the training data set which comprises the historical data.

The method may further comprise training a learning algorithm according to the training data set comprising the historical data.

The training may comprise training the learning algorithm according to the training data set comprising at least one of in vivo historical training data and in vitro historical training data.

The training may comprise training the learning algorithm according to the training data set comprising continuous monitoring historical data.

The training may comprise training the learning algorithm according to the training data set comprising test data from the following group: manufacturing test data, patient test data, personalized patient test data, population test data comprising multiple patient data sets. The training data set may be derived from one or more of such different test data for optimizing the training data set with regard to one or more operation status of the sensor.

The training may comprise training the learning algorithm according to the training data set comprising training data indicative of one or more sensor-related parameter from the following group: current values of the sensor, particularly in the case of a continuous monitoring sensor current values of a working electrode; voltage values of the sensor, particularly in the case of a continuous monitoring sensor voltage values of a counter electrode, or voltage values between the reference electrode and the working electrode; temperature of an environment of the sensor during measurement; sensitivity of the sensor; offset of the sensor; and calibration status of the sensor. In dependence on the operation status which is to be detected, one or more of the sensor-related parameters may be selected. With regard to the calibration status of the sensor, for example, it may indicate when a last calibration has been performed.

The one or more sensor-related parameter may include at least one of non-correlated sensor-related parameters, and correlated sensor-related parameters. Two or more sensor-related parameters may be correlated. In such case, the correlated sensor-related parameters may be selected for detecting the operation status by taking into account all the correlated sensor-related parameters. Differently, in case of non-correlated sensor-related parameters a single one of the non-correlated sensor-related parameters may be selected for detecting an operation status. The non-correlated sensor-related parameters may independently allow for detection of operation status.

The method may further comprise validating the trained learning algorithm according to a validation data set comprising measured continuous monitoring data and / or simulated continuous monitoring data indicative, for the sensor, of at least one of: manufacturing fault status, malfunction status, glycemic indicating status, and anamnestic indicating status.

The method may further comprise at least one of receiving continuous monitoring data comprising compressed monitoring data, and training the learning algorithm according to the training data set comprising compressed training data, wherein the compressed monitoring data and / or the compressed training data are determined by at least one of a linear regression method and a smoothing method. The compressed data may be the result of reduction of the dimension of monitoring data or training data. With regard to the smoothing method, kernel smoothing or spline smoothing models or time series analysis known as such may be applied. In the different stages of compression, the monitoring data / training data may comprise a data (measurement signals) per second, data per minute and / or statistic data including characteristic values such as sensor parameters, variance, noise or rate-of-change.

Continuous monitoring data may be provided by the sensor that is a fully or partially implanted sensor for continuous glucose monitoring (CGM). In general, in the context of CGM, an analyte value or level indicative of a glucose value or level in the blood may be determined. The analyte value may be measured in an interstitial fluid. The measurement may be performed subcutaneously or in vivo. CGM may be implemented as a nearly real-time or quasi-continuous monitoring procedure frequently or automatically providing / updating analyte values without user interaction. In an alternative embodiment, analyte may be measured with a biosensor in a contact lens through the eye fluid or with a biosensor on the skin via trans-dermal measurement in sudor. A CGM sensor may stay in place for several days to weeks and then must be replaced.

With regard to the state machine system, the alternative embodiments described above may apply *mutatis mutandis.*

Description of embodiments

Following, further embodiments are described with reference to figures. In the figures, show:

Fig. 1      an embodiment of a state machine system;
Fig. 2      the flow diagram of an embodiment of the method for determining an operation status for a sensor;
Fig. 3      an overview of data collection for a learning algorithm;
Fig. 4      a Graph of current density measured at the working electrode of a sensor;
Fig. 5      an error-free measurement;
Fig. 6      a measurement exhibiting a fluidics error;
Fig. 7      a measurement exhibiting a maxed out current error;
Fig. 8      the degree of correlation between different parameters used with a learning algorithm;
Fig. 9      an illustration of the adaptation of model characteristics of a random forest model using hyper parameters;
Fig. 10     an illustration of the prediction error of logistic regression;
Fig. 11     a Receiver-Operating-Characteristic-Curve for a logistic regression;
Fig. 12     an example of a tree for a random forest model;
Fig. 13     an exemplary illustration of error for a random forest;
Fig. 14     a comparison of accuracy of different exemplary learning algorithms.

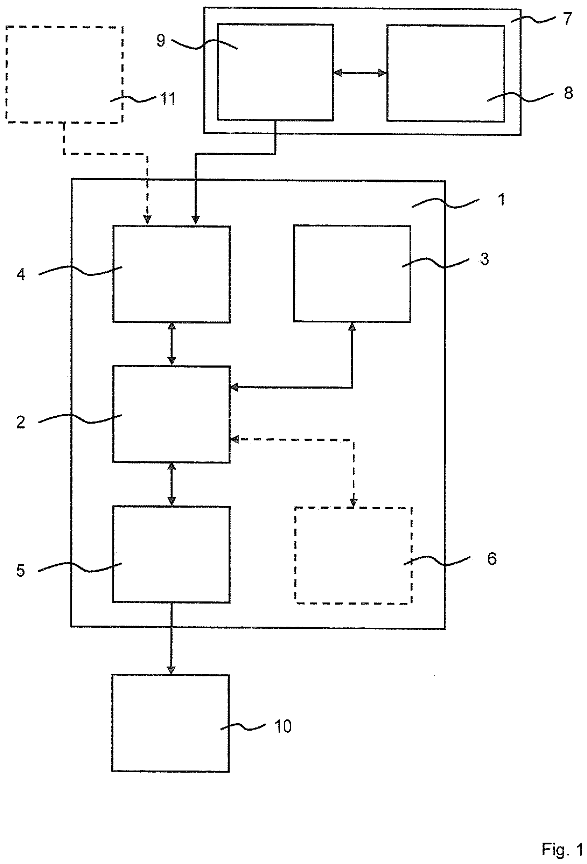

Fig. 1 shows one embodiment of a state machine system 1, which may also be referred to as state analyzing system. The state machine system comprises one or more processors 2, a memory 3, an input interface 4 and an output interface 5. In the shown embodiment, input interface 4 and output interface 5 are provided as separate modules. Alternatively, both input interface 4 and output interface 5 may be integrated in a single module.

In a further embodiment, additional functional elements 7 may be provided in the state machine system 1.

Continuous monitoring data related to an operation of a sensor 7 is received in the one or more processors 2 via the input interface 4. Sensor 7 may be connected to input interface 4 of state machine system 1 via a wire. Alternatively or additionally, a wireless connection, such as Bluetooth, WiFi or other wireless technology, may be provided.

In the embodiment shown, sensor 7 comprises a sensing element 8 and sensor electronics 9. In this embodiment, sensing element 8 and sensor electronics 9 are provided in the same housing of sensor 7. Alternatively, sensing element 8 and sensor electronics 9 may be provided separately and may be connected using a wire and / or wirelessly.

In one embodiment, continuous monitoring data may be provided by a sensor 7 that is a fully or partially implanted sensor for continuous glucose monitoring (CGM). In general, in the context of CGM, an analyte value or level indicative of a glucose value or level in the blood may be determined. The analyte value may be measured in an interstitial fluid. The measurement may be performed subcutaneously or in vivo. CGM may be implemented as a nearly real-time or quasi-continuous monitoring procedure frequently or automatically providing / updating analyte values without user interaction. In an alternative embodiment, analyte may be measured with a biosensor in a contact lens through the eye fluid or with a biosensor on the skin via trans-dermal measurement in sudor.

A CGM sensor may stay in place for several days to weeks and then must be replaced. A transmitter may be used to send information about an analyte value or level indicative of the glucose level via wireless and / or wired data transmission from the sensor to a receiver such as sensor electronics 9 or input interface 4.

Via the output interface 5, output data indicating the detected operation status for the sensor 7 is provided to one or more output devices 10. Any suitable output device may serve as output device 10 is contemplated. For example, output device 10 may comprise a display device. Alternatively or additionally, output device 10 may comprise an alert generator, a data network and / or one or more further processing devices. In another embodiment (not shown), more than one output device 10 is provided.

The one or more output devices 10 may be connected to output interface 5 of state machine system 1 via a wire. Alternatively or additionally, a wireless connection, such as Bluetooth, WiFi or other wireless technology, may be provided.

In an alternative embodiment, the output device 10, or one of the more than one output devices 10, is integrated in state machine system 1.

In an embodiment, one or more further input devices 11 are connected to the input interface 4. Such further input devices 11 may include one or more further sensors to collect training data and / or validation data for use with the learning algorithm. Further input devices 11 may also include, in addition or as an alternative, sensors for acquiring different types of data. An example of such a different type of data is temperature data. Sensor data of such different type of data may be additionally analyzed for detecting an operation status for the sensor 7. In addition or as an alternative, sensor data of such different type of data may be used as training data and / or validation data. Alternatively or additionally, the one or more further input devices 11 may include a data network, external data storage device, user input device, such as a keyboard, mouse or the like, one or more further processing devices and / or any other device suitable to provide relevant data to state machine system 1.

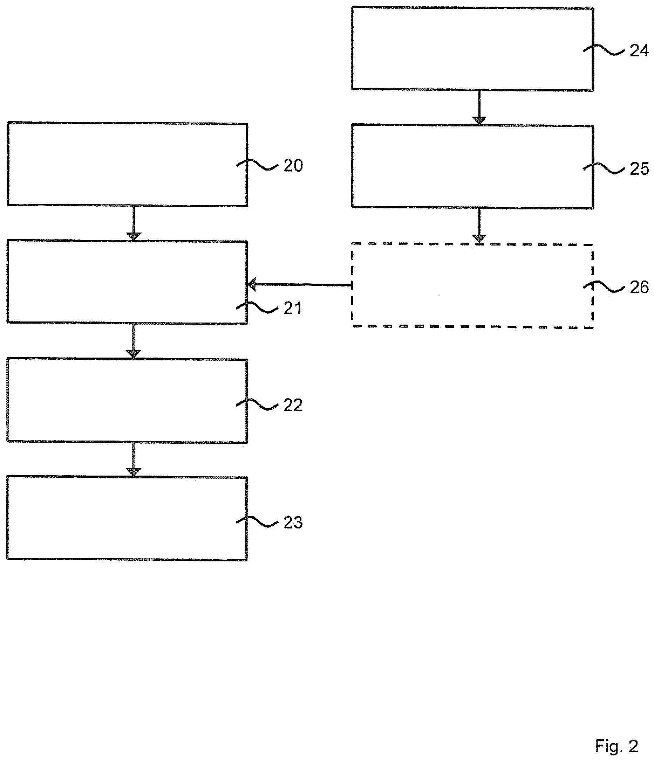

Fig. 2 is a flow diagram illustrating one embodiment of the method for detecting an operation status for a sensor.

In step 20, continuous monitoring data related to an operation of a sensor 6 is received in an input interface 4 of a state machine system 1.

Continuous monitoring data may be indicative of one or more sensor-related parameter. Such sensor-related parameters may include current values of a working electrode of the sensor, voltage values of a counter electrode of the sensor, voltage values between the reference electrode and the working electrode, temperature of an environment of the sensor during measurement, sensitivity of the sensor, offset, and / or calibration status of the sensor. Sensor-related parameters may include non-correlated sensor-related parameters, correlated sensor parameters or a combination thereof.

In one embodiment, continuous monitoring data comprises compressed monitoring data.

In this case, compressed monitoring data is determined by at least one of a linear regression method and a smoothing method.

In step 21, a trained learning algorithm is provided. The learning algorithm is trained according to a training data set comprising historical data. The trained learning algorithm may be provided in the memory 3 of the state machine system 1. Alternatively, the trained learning algorithm may be provided in the one or more processors 2 from the memory 3. In an alternative embodiment, the trained learning algorithm is provided via the input interface 4. For example, the trained learning algorithm may be received from an external storage device. In further embodiments, the trained learning algorithm may be provided in one or more additional functional elements 7 or may be provided in the one more processors 2 from one or more additional functional elements 7.

The order of steps 20 and 21 may be reversed in different embodiments. In a particular embodiment, the trained learning algorithm is provided sensor 7 is put into operation. As a further alternative, step 20 and 21 may be performed, in whole or partially, at the same time.

In step 22, using the one or more processors 2, the continuous monitoring data is analyzed with the trained learning algorithm. In embodiments, in which the trained learning algorithm is not provided in the processor 2, the processor 2 may access the trained learning algorithm to analyze the continuous monitoring data. By analyzing the continuous monitoring data, an operation status for the sensor 7 is detected.

The operation status detected for the sensor in step 22 may be one of several different states. For example, a manufacturing fault status for the sensor indicative of a fault in a process for manufacturing the sensor, a malfunction status for the sensor indicative of a malfunction of the sensor, an anomaly status for the sensor indicative of an anomaly in operation of the sensor, a glycemic indicating status for the sensor indicative of a glycemic index for a patient for whom the continuous monitoring data are provided, and / or an anamnestic indicating status for the sensor indicative of an anamnestic patient status for the patient for whom the continuous monitoring data are provided may be detected.

Following, in step 23, output data indicating the detected operation status for the sensor is provided at output interface 5.

In an embodiment, the method for detecting an operation status for a sensor may further comprise training a learning algorithm according to a training data set comprising historical data.

Still referring to Fig. 2, in step 24, a training data set comprising historical data is provided.

Historical training data may comprise *in vivo* historical training data being indicative of sensor-related parameters acquired while sensor 7 is in operation on a living subject. Alternatively or additionally, historical training data may comprise *in vitro* historical training data being indicative of sensor-related parameters acquired while sensor 7 is not in operation on a living subject.

The training data set provided in step 24 may comprise continuous monitoring historical data.

The training data set may comprise manufacturing test data, patient test data, personalized patient test data and / or population test data comprising multiple patient datasets.

Training data may be indicative of one or more sensor-related parameter. Such sensor-related parameters may include current values of a working electrode of the sensor, voltage values of a counter electrode of the sensor, voltage values between the reference electrode and the working electrode, temperature of an environment of the sensor during measurement, sensitivity of the sensor, offset, and / or calibration status of the sensor. Sensor-related parameters may include non-correlated sensor-related parameters, correlated sensor parameters or a combination thereof.

In one embodiment, the training data set comprises compressed training data. In this case, compressed training data is determined by at least one of a linear regression method and a smoothing method.

In step 25, the learning algorithm is trained according to the training data set provided in step 24.

The learning algorithm may be selected from suitable algorithms. Such learning algorithms include: K-nearest neighbor, support vector machines, naive bayes, decision trees such as random forest, logistic regression such as multinominal logistic regression, neuronal network, decision trees and bayes network. A learning algorithm may be selected based on suitability for use with the continuous monitoring data analyzed in step 22.

Training of the learning algorithm in step 25 may take place in state machine system 1. In this case, in step 24, the training data set may be provided in the memory 3 of the state machine system 1. Alternatively, the training data set may be provided in the one or more processors 2 from the memory 3. In an alternative embodiment, the training data set is provided via the input interface 4. For example, the training data set may be received from an external storage device. In further embodiments, the training data set may be provided in one or more additional functional elements 7 or may be provided in the one more processors 2 and / or the memory 3 from one or more additional functional elements 7.

In an alternative embodiment, training of the learning algorithm in step 25 may take place outside state machine system 1. In this embodiment, in step 24, the training data set is provided in any suitable way that enables training of the learning algorithm.

A further embodiment may include step 26 in which the trained learning algorithm is validated according to a validation data set. The validation data set comprises measured continuous monitoring data and / or simulated continuous monitoring data. This data is indicative, for the sensor, of at least one of: manufacturing fault status, malfunction status, glycemic indicating status, and anamnestic indicating status.

Validating of the trained learning algorithm in step 26 may take place in state machine system 1. In this case, the validation data set may be provided in the memory 3 of the state machine system 1. Alternatively, the validation data set may be provided in the one or more processors 2 from the memory 3. In an alternative embodiment, the validation data set is provided via the input interface 4. For example, the validation data set may be received from an external storage device. In further embodiments, the validation data set may be provided in one or more additional functional elements 7 or may be provided in the one more processors 2 and / or the memory 3 from one or more additional functional elements 7.

In an alternative embodiment, validation of the trained learning algorithm in step 26 may take place outside state machine system 1. In this embodiment, the validation data set is provided in any suitable way that enables validating the learning algorithm.

In one embodiment, the validation data set comprises compressed validation data. In this case, compressed validation data is determined by at least one of a linear regression method and a smoothing method.

Following, additional aspects are described.

Measurements for collecting continuous monitoring data are performed with a plurality of continuous glucose monitoring sensors.

Based on an established sequence of working steps in the field of data mining (compare Shmueli et al., Data Mining for Business analytics - Concepts, Techniques, and Applications with XLMiner, 3rd Ed., New York: John Wiley & Sons, 2016), which is to serve as support for the development of a model, the following steps, all or in part, may be realized:

1. Draw up the problem
2. Obtain data
3. Analyze and clean data
4. Reduce the dimensions, if necessary
5. Specify the problem (classification, clustering, prediction)
6. Share the data in training. Validate and test data set.
7. Select the data mining technique (regression, neuronal network, etc.)
8. Different versions of the algorithm (different variables)
9. Interpret the results
10. Incorporate model into the existing system

Following, a process for data collection is described, which may be applied in an alternative embodiment.

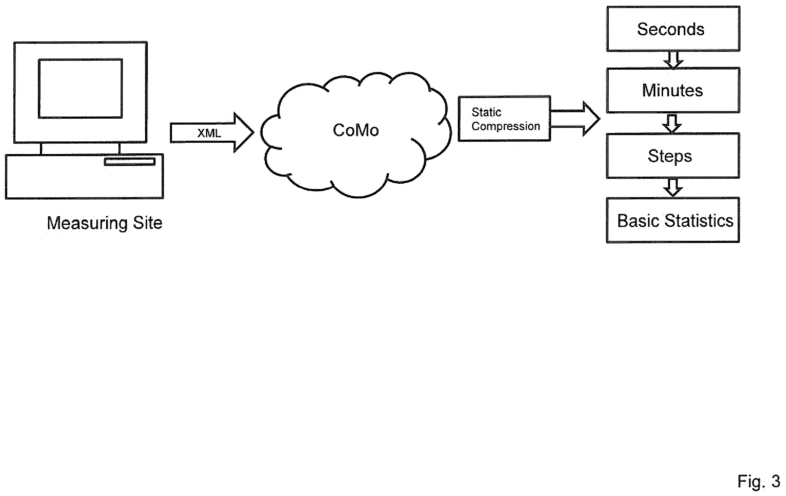

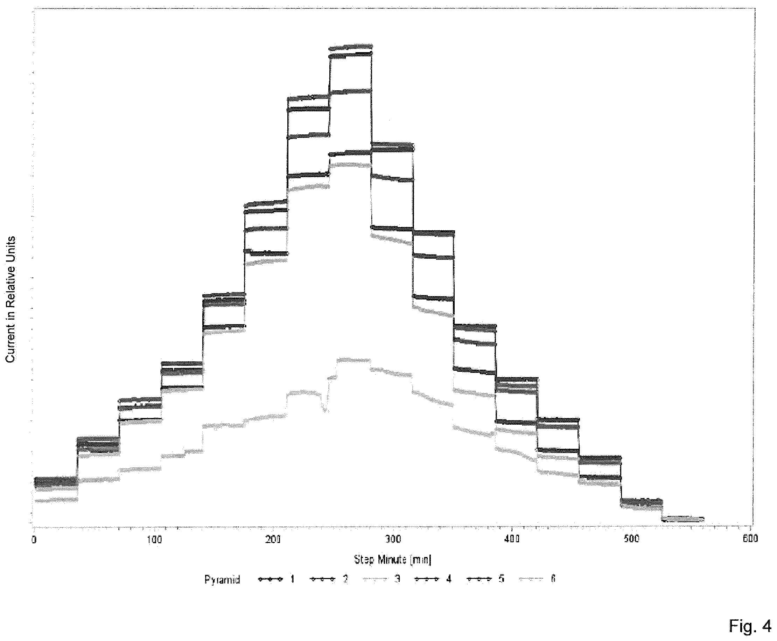

At test sites, the current value of a working electrode of the sensor, the voltage value of the counter electrode of the sensor, the voltage values between the reference electrode and the working electrode may be recorded each second each channel. The temperature of the solution, in which the sensors are located, may be detected each minute. These parameters may be stored in an Extensible Markup Language (XML) file. CoMo, a data processing program, then captures the XML file and provides it as a so-called experiment in the form of an SAS data set. At the lowest stage, this experiment consists of data referring to one second. As shown in Fig. 3, this data is compressed into minute values by means of CoMo. In this step, descriptive statistics are additionally generated, e.g. with minimum, average value and maximum per minute. A compression into step values then takes place. The steps can be observed in the pyramid shape as illustrated in Fig. 4. The last compression stage, the Basic Statistics, corresponds to a characteristic value report per sensor.

To start, data from the highest compression stage, the basic statistics, may be used because access to more complex data may be reserved to cases in which the classification using simpler data provides insufficient results. In addition, the classification of time-resolved data, as they are present in the minute and second stage, would require a different programming language, such as Python.

A plurality of test series, such as 16 test series, were identified, which are distributed to the test sites, resulting, multiplied by the plurality of channels, in one example in 256 data entries.

For the error identification of each sensor, the graphic illustration according to Fig. 4 of the current intensity at the working electrode per minute for each channel is considered. For a measurement for seven days, every day is represented as separate curve. Due to the fact that the sensors run through one day of preparation in the form of a preswelling, only six days are illustrated. It becomes clear from Fig. 4 that on day three, channel 4 differs significantly from the other days and thus no longer follows the typical pyramid shape. Therefore, channel 4 is identified as being faulty.

Once all channels have been analyzed and identified, the test series may be exported from SAS to a memory. In a last step, the test series may be read in R from this memory and stored as reference.

The entire data set was divided into three parts, a training data set, a validation data set as well as a test data set representing continuous monitoring data.

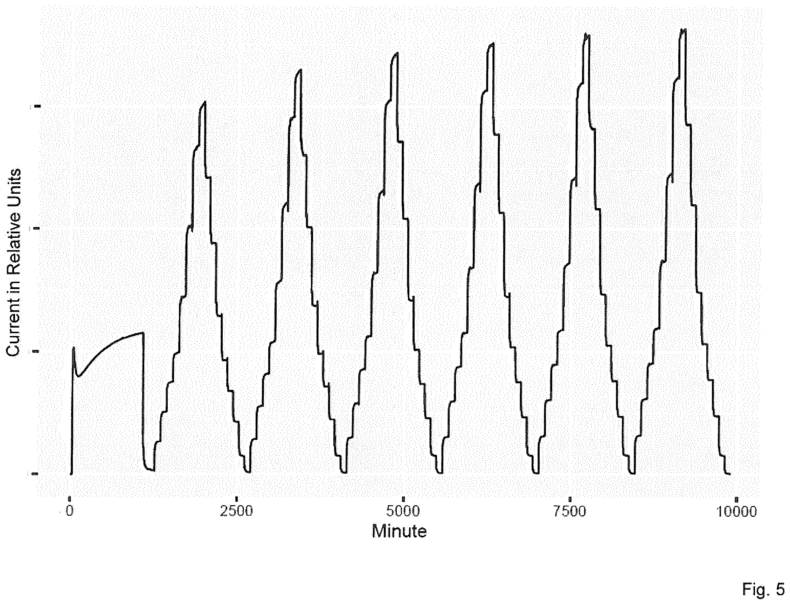

In an alternative embodiment, two types of errors, representing an operation status of the sensor, are to be identified by the models. These are a fluidics error and a maxed out current error. A channel without errors, as shown in Fig. 5, may initially be considered as reference. As in Fig. 4, a pyramid shape can be observed. However, the days are not graphically superimposed, but are arranged in series. Since whether a channel is identified as being faulty is decided by means of the current intensity, the current intensity is also used for the analysis regarding individual errors.

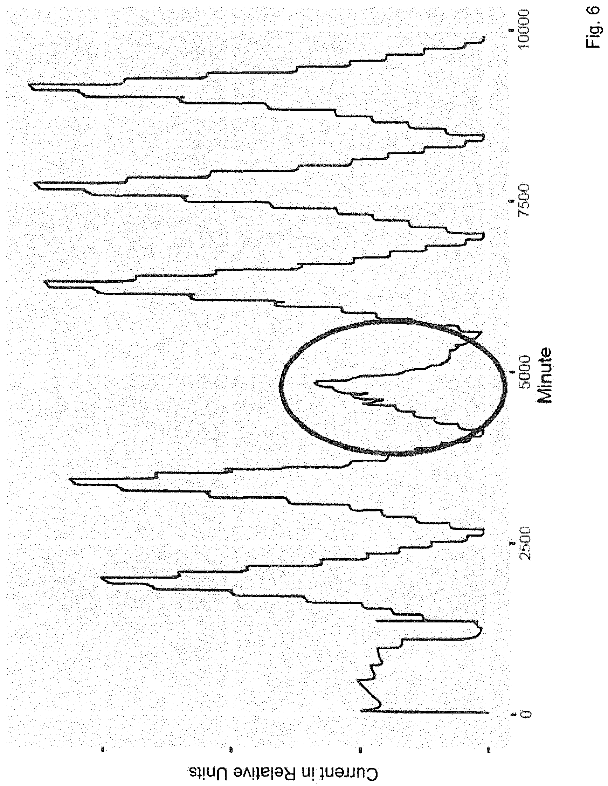

In this embodiment, the fluidics error is in the focus of error detection. Therefore, data from a period of time with a high volume of these defects is chosen. One difficulty associated with this error type is the large variety of manifestations in which it may occur. However, as illustrated in Fig. 6, it can be observed that measured values tend to decrease. The cause for this error lies in the test site unit, which is why this defect may also be referred to as a test site error. Presumably, the cause for this are air bubbles in the test system, which can be caused by temperature fluctuations, for example. Air bubbles in the liquid may form due to a pause in inflow.

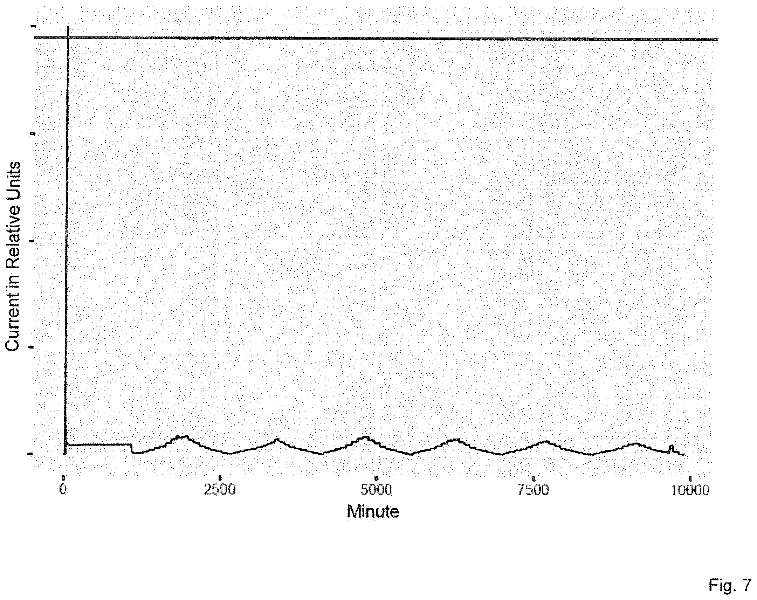

The maxed out current error can appear, when the sensor is inserted into the channel at the beginning of the test. The sensor at the test site is marked with the error type when a current above a threshold value is detected. It is now possible for a member of the staff at the test site to insert the sensor into the channel anew, thus fixing the error. Alternatively, the sensor may ultimately be marked as being faulty. Fig. 7 shows a typical maxed out current error. Compared to Fig. 6, a significantly higher value of the current can be identified at the beginning of the measurement.

In order to be able to mark the data in a meaningful manner, the individual errors may be provided with different error codes according to table 1.

Table 1:

| Error Code | Meaning |
| --- | --- |
| 0 | No Error |
| 1 | Fluidics Error |
| 3 | Maxed out Current Error |
| 99 | Other Error |

## Analysis of Parameters

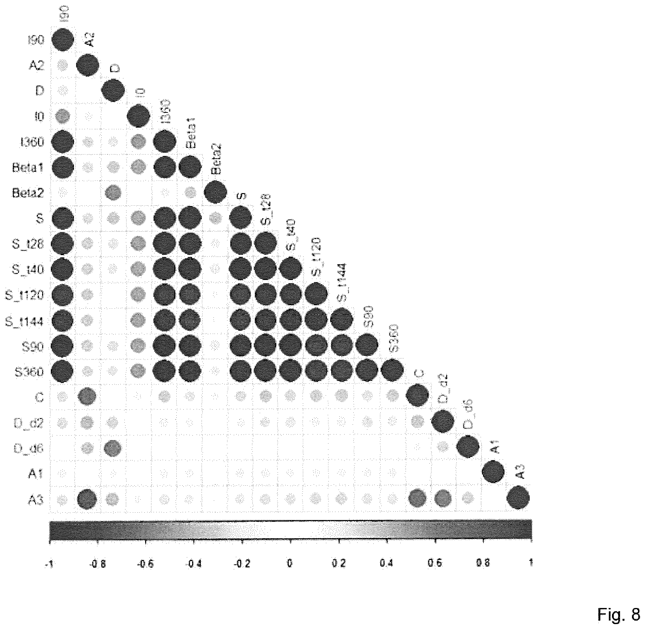

In an alternative embodiment, the strength of the linear connection between the variables may be determined by means of the correlation coefficient, which can have values of between -1 and 1. In the case of a value of 1, a high positive linear correlation is present. When looking at Fig. 8, it can be seen that the parameter S360 correlates with a very large number of other parameters.

As indicated above, there may be variables, such as the current, which may be measured directly at the test site. In an embodiment, when compressing the data, a linear model as well as a spline model are used, which estimate various parameters. Due to the fact that the data set, which is to be used later, includes compressed data, integrated models are considered.

## Measured Values

The analysis of the normal distribution condition, which, according to DIN 53804-1 can be carried out graphically by means of Quantil-Quantil plots, may be of interest for the descriptive statistics regarding the measured values representing sensor-related parameters. The X-axis of a QQPlot is defined by the theoretical quantile, and the Y-axis is defined by the empirical quantile. A normally distributed parameter results in a straight line, which is illustrated as straight line in the QQPlot. In addition, there are various normal distribution tests, such as the Chi-square test or the Shapiro-Wilk test. These hypotheses tests define the null hypothesis as a presence of the normal distribution and the alternative hypothesis, in contrast, assumes that a normal distribution is not present. These test methods are highly sensible with respect to deviations. In an embodiment, normal distribution may therefore be analyzed by means of QQPlot for each parameter.

Measured values may include the sensor current for different glucose concentrations. These may be determined as certain time period medians and may, additionally or alternatively, be averaged. Measured values may further include the sensitivity of the sensor. Additionally or alternatively, measured values may include parameters characteristic of the graphs that describe measured values, such as the sensor current. These may, for example, include a drift and / or a curvature. In addition or as an alternative, values may include statistical values regarding other measured values. Measured values may be approximated employing different models, such as a linear model and / or a spline model. All or any of the measured values and parameters may be determined at different glucose concentrations and / or for different time periods.

## Learning algorithms

In an alternative embodiment, several modeling methods for a learning algorithm are chosen (see, for example, Domingos, A Few Useful Things to Know About MachineLearning, Commun. ACM 55.10, S. 78-87. DOI: 10.1145/2347736.2347755, 2012) and are analyzed with regard to their advantages as well as disadvantages. In addition, the methods may be analyzed with regard to their compatibility with regard to the problem, in order to be able to make a method selection. Following, exemplary methods are described (Sammut et al., Encyclopedia of Machine Learning, 1st. Springer Publishing Company, Incorporated, 2011). Table 2 summarizes advantages and disadvantages of the methods.

## K-nearest neighbor

The goal of this method is to classify an object into a class, into which similar objects of the training quantity have already been classified, whereby the class which appears most frequently is output as result. In order to determine the proximity of the objects, a similarity measure, such as, for example, the Euclidian distance, is used. This method is very well suited for significantly larger data quantities, which are not present in the present example. This is also why this model is not taken into the comparative consideration.

Support Vector Machines

In this method, a hyper plane is calculated, which classifies objects into classes. For calculating the hyper plane, the distance around the class boundaries is to be maximized, which is why the Support Vector Machine is one of the 'Large Margin Classifiers'. An important assumption of this method is the linear separability of the data, which, however, can be expanded to higher dimensional vector spaces by means of the Kernel trick. Large data quantities, which in some embodiments are not present, are required for a classification with less overfitting.

Naive Bayes

The naive assumption is that the present variables are statistically independent from one another. This assumption is not true for most cases. In many cases, Naive Bayes nonetheless reaches good results to the effect that a high rate of correct classifications is reached, even if the attributes correlate slightly. Naive Bayes is characterized by a simple mode of operation and may thus be adopted into the model selection.

Logistic regression

In connection with the logistic regression, a likelihood is calculated for the analysis as to what extent the characteristic of a dependent variable can be attributed to values of independent variables.

Neuronal Networks

Artificial neuronal networks are based on the biological structure of neurons in the brain. A simple neuronal network consists of neurons arranged in three layers. These layers are the input layer, the hidden layer and the output layer. Between the layers, all neurons are connected to one another via weights, which are optimized step by step in the training phase. Neuronal networks are currently used heavily in many areas and thus comprise a large spectrum of model variations. There is a plurality of hyper parameters, which must be determined from experience values for the optimization of such networks. In some embodiments, for reasons of time efficiency, these hyper parameters are not determined.

Decision Trees

Decision trees are sorted, layered trees, which are characterized by their simple and easily comprehensible appearance. Nodes which are located close to the root are more significant for the classification than nodes located close to the leaf. In one embodiment, due to the fact that decision trees often experience problems caused by overfitting, the methodology of the random forest is chosen for the model selection. This method consists of a plurality of decision trees, whereby each tree represents a partial quantity of variables.

Bayes Networks

A Bayes network is a directed graph, which illustrates multi-variable likelihood distributions. The nodes of the network correspond to random variables and the edges show the relationships between them. A possible application can be in diagnostics to illustrate the cause of symptoms of a disease. For developing a Bayes network, it is essential to be able to describe the dependencies between the variables in as much detail as possible. For the errors addressed in some embodiments, the generation of such a graph is not feasible.

Table 2:

| Method | Advantage | Disadvantage |
| --- | --- | --- |
| K-nearest Neighbor | Learning phase is practically nonexistent as all training data is only temporarily stored and only evaluated when there are new objects to classify ('lazy learning'). | Finding nearest neighbor makes classification phase very complex and slow for large quantities of data. |
| Support Vector Machines | Special variables allow for falsely assigning single data points, avoiding over-fitting. | Large quantities of data are needed for a classification with as little over-fitting as possible |

(continued)

| Method | Advantage | Disadvantage |
|---|---|---|
| Naive Bayes | Reaches high accuracy and a speed comparable to Decision Tree methods and Neuronal Networks when applied to large quantities of data. Training time is linear with respect to quantity of data and number of attributes. | Data must be normally distributed, otherwise, model is not precise. |
| Logistic Regression | For classification, non-relevant variables may be identified easily using Backwards-Elimination. | Modelling may be more difficult when many interrelations exist between variables. |
| Decision Trees | Decision Trees may easily be transformed into interpretable decision rules, following all paths from root to leaf nodes. Variables that are occur close to the root node due to high relevancy for classification allow a prioritization of the variables. | Variance is often large. Therefore, trees should trimmed. |
| Neuronal Networks | Neuronal Networks can illustrate very complex problems over a large range of parameters in the form of weight matrices. | A high number of hyper parameters exists, that need to be set based on experience for the optimization of such Networks. The training phase is very long when the number of variables is high. |
| Bayes Networks | A Bayes Network may be displayed in the form of a graph. | Probabilities for parameters have to be estimated, necessitating experts. Distribution of random variables may be difficult for more complex data, as e.g. child nodes may follow a Bernoulli distribution while parent nodes follow a Gauss distribution. |

Method Selection

In an alternative embodiment, models are initially considered theoretically and are analyzed with regard to their assumptions, whereupon the first implementation takes place, which may then be optimized by means of various methods.

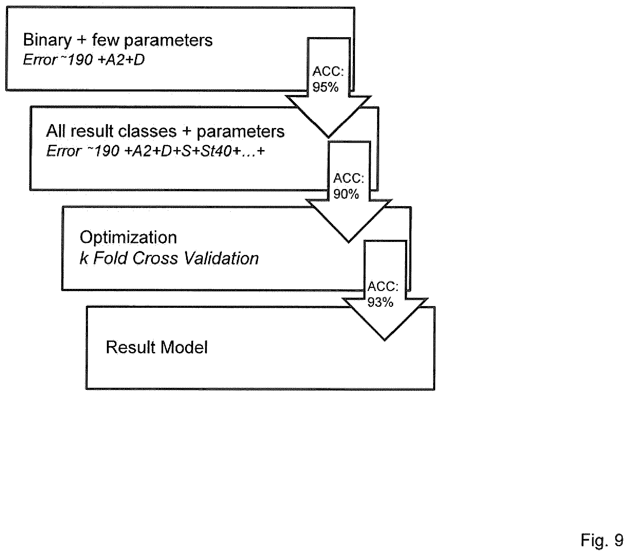

In the first step, a binary problem with a linear model may be used, which includes three variables of the total quantity. The learning algorithms represented by the models may be subsequently trained with all classes and parameters, based on the actual problem. Finally, an adaptation of the model characteristics with regard to the data at hand may be made by means of hyper parameters such as, for example, the number of the decision trees in the case of Random Forest. An illustration with regard to this process using the example of the Random Forest model is illustrated in Fig. 9. The abbreviation ACC identifies the accuracy, which decreases with the first adaptation, but which then improves again with the optimization step by means of cross validation.

Naive Bayes:

This model, which may be used in an embodiment, is based on Bayes' theorem and may serve as a simple and quick method for classifying data. In such an embodiment, it is a condition that the data present is statistically independent from one another and that it is distributed normally. Due to the fact that the method can determine the relative frequencies of the data in only a single pass, it is considered to be a simple as well as quick method.

According to Bayes' theorem, the following formula serves to calculate conditional likelihoods:

$$P(y \mid x) = \frac{P(x \mid y)\, P(y)}{P(x)}$$

When assuming that the attributes are present independently from one another, the Naive Bayes classifier can be defined as follows:

$$\mathrm{pred}(x) = \mathrm{arg\,max}\, P(y) \prod_{i=1}^{n} P(x_i \mid y)$$

This function always predicts the most likely class y for an attribute $x_i$ with the help of the maximum a posteriori rule. The latter behaves similar to the maximum likelihood method, but with the knowledge of the a priori term. When metric data is present in the data set, a distribution function is required in order to calculate the conditional likelihoods for $P(x_i| y)$. In an embodiment, Naive Bayes may also fall back on the normal distribution (Berthold et al., Guide to Intelligent Data Analysis: How to Intelligently Make Sense of Real Data, 1 st, Springer Publishing Company, Incorporated, 2010). In spite of the fact that a normal distribution is not present in the case of many CGM variables, Naive Bayes may be used because it can attain a high rate of correct classifications in spite of slight deviations from normal distribution.

$$P(x_i \mid y) = N(x_i, \mu, \sigma^2)$$

$\mu$ the average value and $\sigma$ the variance are calculated for each attribute xi and each class y.

Due to the fact that a smaller data set is sufficient for a good prediction in the case of this model, only four measurements may be used as input in one embodiment. In one embodiment, for first consideration, a partial quantity of the available parameters, consisting of A2, I90 and D, may be chosen:.
Naive Bayes may be used determining the probability of an error under the condition that I90 appears in one class.

$$P(F \mid I90) = \frac{P(I90 \mid F)\, P(F)}{P(I90)}$$

In one embodiment, no statement is to be made about the type of error. So that a new identification of the data does not need to take place, four test sites may be chosen which contain only fluidic errors. In this case, the error code 0 may be identified as no error and 1 may be identified as error in general. Table 3 illustrates an excerpt of the input data set of one embodiment for Naive Bayes.

Table 3:

|  | 190 | A2 | D | Error |
|---|---|---|---|---|
| 23 | 6.856153 | 2.792434 | 3.721495 | 0 |
| 24 | 6.012486 | 5.013247 | 11.643365 | 1 |
| 25 | 5.687802 | 5.191772 | 10.178749 | 1 |
| 26 | 6.682197 | 2.971844 | 3.807647 | 0 |
| 27 | 4.175271 | 6.464843 | 34.742799 | 1 |

As illustrated in Table 4, the model output may include the calculated a priori values for the classes. In a next step, the average value as well as the standard deviation of each variable for class 0 (no error) and for class 1 (error) may be calculated. They may serve to determine the distribution function of the variable based on the normal distribution.

Table 4:

| 0 | 1 |
|---|---|
| 0.6212121 | 0.3787879 |

The quality of the model may be evaluated by means of various parameters of the output. As illustrated in Table 5, in one embodiment, from this output, the accuracy, the sensitivity and the specificity may be of predominant significance.

Table 5:

| Parameter | Binary | Types of Errors | | | |
|---|---|---|---|---|---|
|  |  | Error 0 | Error 1 | Error 3 | Error 99 |
| Sensitivity | 0.7857 | 0.9298 | 0.9091 | 1.0000 | 0.0000 |
| Specificity | 0.9333 | 0.8750 | 0.9516 | 0.9444 | - |
| Pos.Pred.Value | 0.9166 | 0.9636 | 0.7692 | 0.2000 | 1.00000 |
| Neg.Pred.Value | 0.8235 | 0.7778 | 0.9833 | 1.0000 | 0.94521 |
| Prevalence | 0.4828 | 0.7808 | 0.1507 | 0.0137 | 0.05479 |

(continued)

| Parameter | Binary | Types of Errors | | | |
| | | Error 0 | Error 1 | Error 3 | Error 99 |
| --- | --- | --- | --- | --- | --- |
| Accuracy | 0.8621 | 0.8767 | | | |
| Kappa | 0.7225 | 0.6789 | | | |

In one embodiment, the accuracy allows for a first impression about the results of the models and may thus be used for assessing the quality.

$$Accuracy = \frac{Correctly\ Classified}{Total\ Number}$$

In certain embodiments, in order to be able to assess the significance of the accuracy, the Kappa value may be used. The Kappa value is a statistical measure for the correspondence of two quality parameters, in this embodiment of the observed accuracy with the expected accuracy. After the observed accuracy and the expected accuracy are calculated, the Kappa value can be determined as follows:

$$Kappa = \frac{(Observed\ Accuracy - Expected\ Accuracy)}{(1 - Expected\ Accuracy)}$$

Different approaches exist for the interpretation of the Kappa value. One such approach, known from (Landis et al., The Measurement of Observer Agreement for Categorical Data, Biometrics 33, S. 159-174, 1977), is summarized in table 6:

Table 6:

| Kappa | Interpretation |
| --- | --- |
| < 0 | Bad correspondence |
| 0-0.20 | Some correspondence |
| 0.21-0.40 | Sufficient correspondence |
| 0.41-0.60 | Medium correspondence |
| 0.61-0.80 | Considerable correpondence |
| 0.81-1.00 | Almost complete corrspondence |

In an embodiment, the positive predictive value, negative predictive value, the sensitivity and the specificity may be determined.

The positive predictive value specifies the percentage of the values, which have been correctly classified as being faulty, of all of the results, which have been classified as being faulty (corresponds to the second row of the four-field table).

Accordingly, the negative predictive value specifies the percentage of the values, which have been correctly classified as being free from error, of all of the results, which have been classified as being free from error (corresponds to the second line of the four-field table).

The sensitivity specifies the percentage of the objects, which have been correctly classified as being positive, of the actually positive measurements:
The specificity specifies the percentage of the objects, which have been correctly classified as being negative, of the measurements, which are in fact negative.

In an embodiment, the prediction of the binary model with the variables A2, D and I90 as well as the holistic model can be illustrated via a four-field table. In the embodiment illustrated in table 7, the binary model has the most difficulties in the area of the rate of false negatives, which is reflected in a sensitivity of ≈0.7857.

Table 7:

| | | Reality | |
|---|---|---|---|
| | | 0 | 1 |
| Prediction | 0 | 14 | 3 |
| | 1 | 1 | 11 |

In an alternative embodiment, after naive Bayes has been discussed in the context of a binary question, all error types and variables may then be highlighted at a second stage. The implementation may be based on all of the available data. If the accuracy as well as the Kappa value behave similarly in both model versions, this may reinforce the thesis that Naive Bayes with less data can already reach good results.

Logistic Regression

A logistic regression may be implemented as known as such (Backhaus et al., Multivariate Analysemethoden: Eine anwendungsorientierte Einführung, Springer, Berlin Heidelberg, 2015). Logistic regression may be used to determine a connection between the manifestation of an independent variable and a dependent variable. Normally, the binary dependent variable Y is coded as 0 or 1, i.e. 1: an error is present, 0: no error is present. A possible application of logistic regression in the context of CGM is determining whether current value, spline and sensitivity are connected to the manifestation of an error.

In an embodiment, logistic regression may be implemented using a generalized linear model (see, for example, Dobson, An Introduction to Generalized Linear Models, Second Edition. Chapman & Hall/CRC Texts in Statistical Science, Taylor & Francis, 2010). This may be advantageous as linear models are easily interpreted.

Evaluation:

Table 8 shows a comparison of a simplified model of one embodiment using variables I90, A2 and D to a model using all variables. In this embodiment, accuracy for the model using all variables lies about 7% above accuracy for the simplified model, suggesting that the simplified model does not use the variables relevant for classification.

Table 8:

| Parameter | I90, A2, D | All variables |
|---|---|---|
| Sensitivity | 0.5625 | 0.8750 |
| Specificity | 0.9649 | 0.9649 |
| Pos.Pred.Value | 0.8182 | 0.8750 |
| Neg.Pred.Value | 0.8871 | 0.9649 |
| Prevalence | 0.2192 | 0.2192 |
| Accuracy | 0.8767 | 0.9452 |
| Kappa | 0.5942 | 0.8399 |

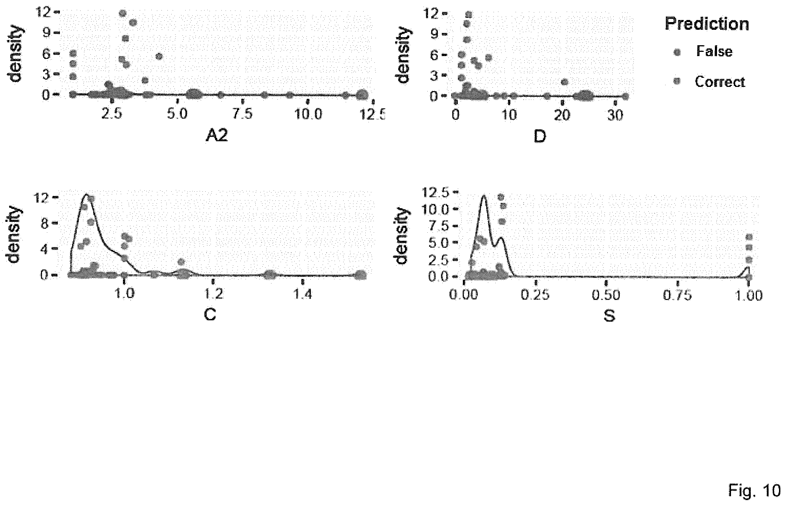

The relevant parameters may be identified using 'backwards elimination' (Sheather, A Modern Approach to Regression with R, Springer Science & Business Media, 2009) and the Akaike information criterion (Aho Ket al., Model selection for ecologists: the worldviews of AIC and BIC, Ecology, 95: 631-636, 2014). These may be examined regarding the prediction error of the logistic regression. Fig. 10 shows, for one embodiment, the distribution density of the variables as well the position of falsely predicted values. Since the latter are present at the edge of the distribution as well as in the area of measurements without error, a correct prediction of all faulty measurements is not possible by simple association rules in this embodiment.

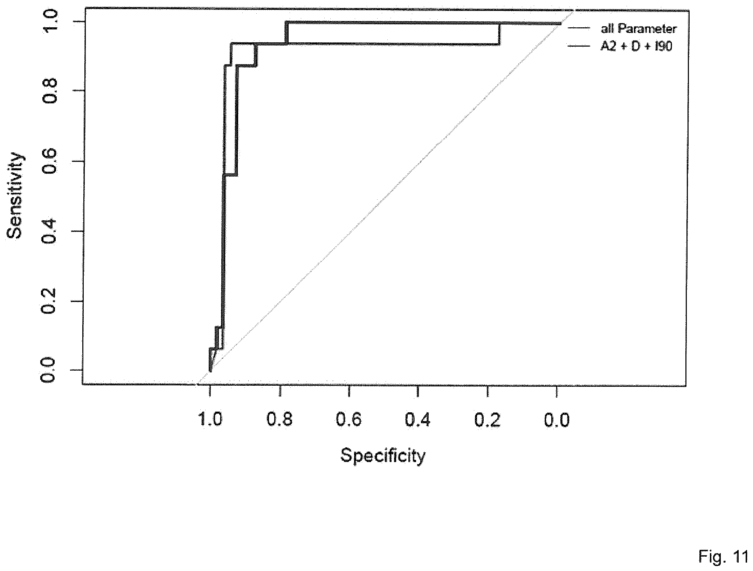

In an embodiment, sensitivity and specificity may be determined using a Receiver-Operating-Characteristic-Curve (ROC). In this case, an ideal curve rises vertically at the start, signifying a rate of error of 0%, with the rate of false positives only rising later. A curve along the diagonal hints at a random process. Fig. 11 shows the ROC for logistic regression for an exemplary embodiment.

Multinomial Logistic Regression:

In a multinomial logistic regression, dependent variable X may have more than two different values, making binary logistic regression a special case of multinomial logistic regression.

Random Forest

Random forest follows the principle of Bagging which states that the combination of a plurality of classification methods increases accuracy of classification by training several classifications with different samples of the data. In an embodiment, a random forest algorithm as known as such (Breiman, Random Forests, Mach. Learn. 45.1, S. 5-32. DOI: 10.1023/A:1010933404324, 2001) may be used.

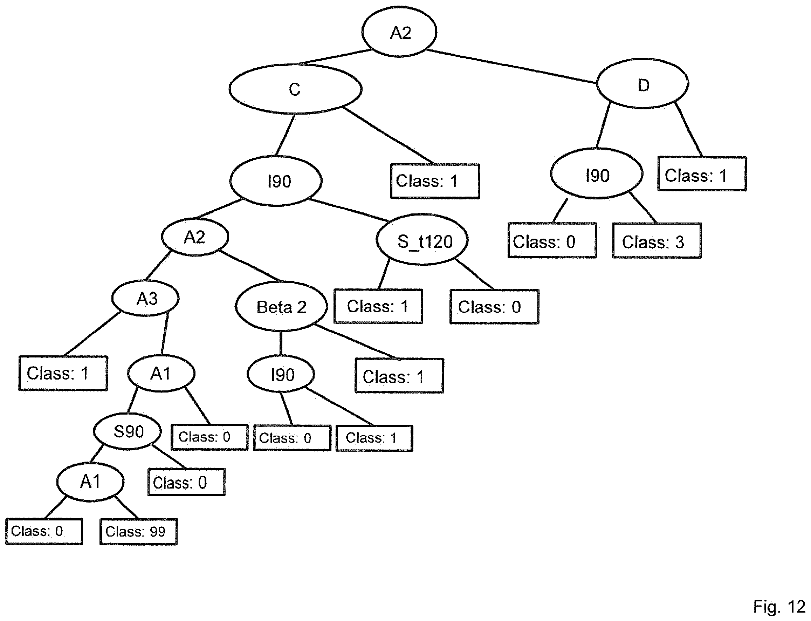

In such embodiment, when a new element is fed to the decision trees, each tree determines a class as a result. In the next step, the resulting class is determined based on the class proposed by the majority of trees. Fig. 12 shows a tree of one exemplary embodiment.

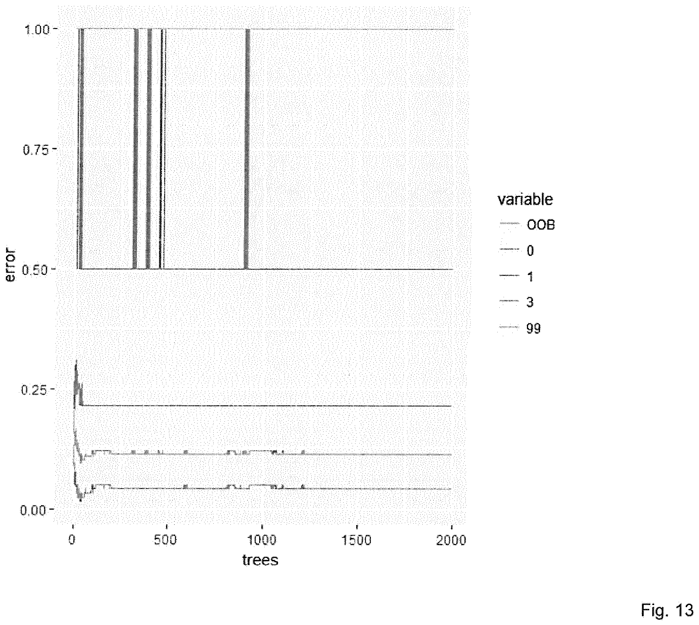

Random forest may be optimized using, for example, the number of trees and / or the number of nodes in a tree. In Fig. 13 an example of error for a random forest is shown for one embodiment, in which the probability of an error regarding the maxed out current error oscillates between 50% and 100%. In this example, all "other errors" are classified falsely as can be seen from the line at the top. This may be due to a small number of occurrences of maxed out current errors and other errors.

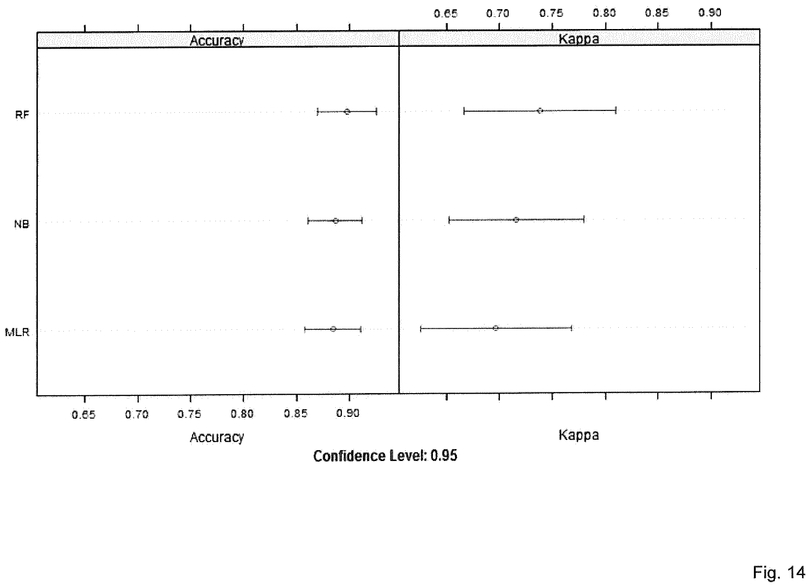

Fig. 14 shows a comparison of accuracy of exemplary learning algorithms of an alternative embodiment: a multinomial logistic regression, a naive bayes and a random forest. On the left, confidence intervals of accuracy are presented. On the right, kappa values of each model are shown.

For this embodiment, the Kappa value allows the assumption of a trend according to which the accuracy of the multi-nominal logistic regression is less significant as compared to the other models.

This assumption is confirmed by the prediction of the trained models for the test data set of this embodiment, which is illustrated in the four-field tables summarized in table 9. The measurements of the test data set were chosen randomly in order to simulate an actual data input. In spite of a maxed out current error not being present in the test data set, the multinominal logistic regression erroneously predicts this error type. However, the model has the most problems with the fluidics error, of which not a single case was classified correctly.

Table 9:

| | Multinomial Logistic Regression | | | | | Naive Bayes | | | | Random Forest | | |
|---|---|---|---|---|---|---|---|---|---|---|---|---|
| | Reality | | | | | Reality | | | | Reality | | |
| | 0 | 1 | 99 | 3 | | 0 | 1 | 99 | | 0 | 1 | 99 |
| Prediction 0 | 37 | 1 | 22 | 0 | Prediction 0 | 34 | 7 | 0 | Prediction 0 | 37 | 10 | 1 |
| 1 | 0 | 0 | 3 | 0 | 1 | 3 | 30 | 1 | 1 | 0 | 32 | 0 |
| 99 | 0 | 0 | 16 | 0 | 99 | 0 | 5 | 0 | 99 | 0 | 0 | 0 |
| 3 | 0 | 0 | 1 | 0 | | | | | | | | |

For this embodiment, the multi-nominal logistic regression thus corresponds to an accuracy of 66% and is thus lower than Naive Bayes with 80% and random forest with 88% of correctly classified cases. The first possible cause for this could be the correlations between the parameters, which can lead to distorted estimates and to increased standard errors. However, Naive Bayes also requires that the parameters do not correlate and this model reaches significantly better results for the embodiment shown. The reason for this could be that Naive Bayes can already reach a high accuracy with very small data quantities. With higher data quantities for the training of the models, the accuracy of Naive Bayes could strongly increase in spite of correlations of the parameters. However, the second assumption of the multinominal logistic regression could be violated as well, the 'Independence of irrelevant alternatives'. This specifies that the odds ratio of two error types is independent from all other response categories. It may be assumed, for example, that the selection of the result class "fluidics error" or "no error" is not influenced by the presence of "other errors".

In an embodiment, the random forest provides the highest rate of correctly classified cases with 86%, whereby a plurality of incorrectly classified cases are predicted as 'no error', even though a fluidics error is present. The reason for the fact that in this embodiment random forest represents the most successful model with regard to the prediction could be, on the one hand, that the tree structure makes it possible to arrange the parameters with respect to their interactions. On the other hand, random forest could be optimized as compared to the multi-nominal logistic regression and Naive Bayes in R without much effort, due to the number of the trees. This may be made possible by means of a graphic of the error relating to the number of decision trees which shows the number of decision trees, at which the error converges.

As an alternative to compressed data which is not covered by the present invention, uncompressed data may be used. For data exhibiting time resolution, it is possible to achieve a prediction using neuronal networks such as recurrent networks. Recurrent neuronal networks have the advantage that no assumptions have to be made prior to the creation of the model.

**Claims**

1. A computer implemented method for detecting an operation status for a continuous glucose measurement sensor (7), the method, in a state machine, comprising

   - receiving continuous monitoring data (20) related to an operation of a sensor and comprising compressed monitoring data;
   - providing a trained learning algorithm (21) for detecting an operation status for the sensor (7) which signifies a sensor function, wherein the learning algorithm is trained according to a training data set comprising historical data, and compressed training data;
   - detecting an operation status for the sensor (7) by analyzing the continuous monitoring data with the trained learning algorithm (22); and
   - providing output data (23) indicating the detected operation status for the sensor (7); wherein
   - the historical data consist of data that are at least one of collected, detected and measured prior to the detecting the operation status; **characterized in that**:
   - the compressed monitoring data and the compressed training data are determined by at least one of a linear regression method and a smoothing method and are the result of reduction of the dimension of monitoring data and training data, respectively, wherein in the different stages of compression, the monitoring data and training data, respectively, comprise a data per second, data per minute and / or statistic data including characteristic values such as sensor parameters, variance, noise or rate-of-change.

2. Method of claim 1, wherein the detecting comprises at least one of

   - detecting a manufacturing fault status for the sensor (7) indicative of a fault in a process for manufacturing the sensor (7);
   - detecting a malfunction status (7) for the sensor indicative of a malfunction of the sensor (7);
   - detecting an anomaly status for the sensor indicative of an anomaly in operation of the sensor (7); and - detecting an anamnestic indicating status for the sensor (7) indicative of an anamnestic patient status for the patient for whom the continuous monitoring data are provided.

3. Method of claim 1 or 2, wherein providing the trained learning algorithm (21) comprises providing at least one learning algorithm selected from the following group

   - K-nearest neighbor;
   - support vector machines;
   - naive bayes;
   - decision trees such as random forest;
   - logistic regression such as multinominal logistic regression;
   - neuronal network;
   - decision trees; and
   - bayes network.

4. Method of at least one of the preceding claims, further comprising training a learning algorithm according to the training data set comprising the historical data (25).

5. Method of claim 4, wherein the training (25) comprises training the learning algorithm according to the training data set comprising at least one of *in vivo* historical training data and *in vitro* historical training data.

6. Method of claim 4 and 5, wherein the training (25) comprises training the learning algorithm according to the training data set comprising continuous monitoring historical data.

7. Method of at least one of claims 4 to 6, wherein the training (25) comprises training the learning algorithm according to the training data set comprising test data from the following group: manufacturing test data, patient test data, personalized patient test data, population test data comprising multiple patient datasets.

8. Method of at least one of claims 4 to 7, wherein the training (25) comprises training the learning algorithm according to the training data set comprising training data indicative of one or more sensor-related parameter from the following group: current values of the sensor (7), particularly in the case of a continuous monitoring sensor current values of a working electrode; voltage values of the sensor (7) or voltage values between the reference electrode and the working electrode; temperature of an environment of the sensor (7) during measurement; sensitivity of the sensor (7); offset of the sensor (7); and calibration status of the sensor (7).

9. Method of claim 8, wherein the one or more sensor-related parameter include at least one of

   - non-correlated sensor-related parameters; and
   - correlated sensor-related parameters.

10. Method of at least one of the preceding claims, as far as referring to claim 2, further comprising validating the trained learning algorithm according to a validation data set (26) comprising measured continuous monitoring data and / or simulated continuous monitoring data indicative, for the sensor (7), of at least one of: manufacturing fault status, malfunction status, and anamnestic indicating status.

11. Method of at least one of the preceding claims, further comprising

   - validating the trained learning algorithm according to the validation data set comprising compressed validation data;

   wherein the compressed validation data are determined by at least one of a linear regression method and a smoothing method.

12. A state machine system (1), having one or more processors (2) configured for data processing and for performing a method for detecting an operation status for a continuous glucose measurement sensor (7), the method comprising

   - receiving continuous monitoring data (20) related to an operation of a sensor and comprising compressed monitoring data;
   - providing a trained learning algorithm (21) for detecting an operation status for the sensor (7) which signifies a sensor function, wherein the learning algorithm is trained according to a training data set comprising historical data, and compressed training data;
   - detecting an operation status for the sensor (7) by analyzing the continuous monitoring data with the trained learning algorithm (22); and
   - providing output data (23) indicating the detected operation status for the sensor (7).; wherein
   - the historical data consist of data that are at least one of collected, detected and measured prior to the detecting the operation status; **characterized in that**:
   - the compressed monitoring data and the compressed training data are determined by at least one of a linear regression method and a smoothing method and are the result of reduction of the dimension of monitoring data and training data, respectively, wherein in the different stages of compression, the monitoring data and training data, respectively, comprise a data per second, data per minute and / or statistic data including characteristic values such as sensor parameters, variance, noise or rate-of-change.

**Patentansprüche**

1. Computerimplementiertes Verfahren zum Detektieren eines Betriebsstatus für einen Sensor (7) zur kontinuierlichen Glukosemessung, wobei das Verfahren in einer Zustandsmaschine Folgendes umfasst:

- Empfangen von kontinuierlichen Überwachungsdaten (20), die sich auf einen Betrieb eines Sensors beziehen und komprimierte Überwachungsdaten umfassen;
- Bereitstellen eines trainierten Lernalgorithmus (21) zum Detektieren eines Betriebsstatus für den Sensor (7), der eine Sensorfunktion angibt, wobei der Lernalgorithmus gemäß einem Trainingsdatensatz trainiert wird, der historische Daten und komprimierte Trainingsdaten umfasst;
- Detektieren eines Betriebsstatus für den Sensor (7) durch Analysieren der kontinuierlichen Überwachungsdaten mit dem trainierten Lernalgorithmus (22);
und
- Bereitstellen von Ausgabedaten (23), die den detektierten Betriebsstatus für den Sensor (7) angeben;
wobei
- die historischen Daten aus Daten bestehen, die vor dem Detektieren des Betriebsstatus mindestens eines von gesammelt, detektiert und gemessen sind;

**dadurch gekennzeichnet, dass**:

- die komprimierten Überwachungsdaten und die komprimierten Trainingsdaten durch mindestens eines von einem linearen Regressionsverfahren und einem Glättungsverfahren bestimmt werden und das Ergebnis einer Reduktion der Dimension der Überwachungsdaten bzw. Trainingsdaten sind, wobei in den verschiedenen Stufen der Komprimierung die Überwachungsdaten bzw. Trainingsdaten Daten pro Sekunde, Daten pro Minute und/oder statistische Daten, die charakteristische Werte wie Sensorparameter, Varianz, Rauschen oder Änderungsrate einschließen, umfassen.

2. Verfahren nach Anspruch 1, wobei das Detektieren mindestens eines von Folgendem umfasst

- Detektieren eines Herstellungsfehlerstatus für den Sensor (7), der einen Fehler in einem Prozess zum Herstellen des Sensors (7) angibt;
- Detektieren eines Fehlfunktionsstatus für den Sensor (7), der eine Fehlfunktion des Sensors (7) angibt;
- Detektieren eines Anomaliestatus für den Sensor, der eine Anomalie im Betrieb des Sensors (7) angibt; und
- Detektieren eines Anamneseanzeigestatus für den Sensor (7), der einen Patientenanamnesestatus für den Patienten, für den die kontinuierlichen Überwachungsdaten bereitgestellt werden, angibt.

3. Verfahren nach Anspruch 1 oder 2, wobei das Bereitstellen des trainierten Lernalgorithmus (21) das Bereitstellen mindestens eines Lernalgorithmus umfasst, der aus der folgenden Gruppe ausgewählt ist

- K-nächster Nachbar;
- Support Vector Machines;
- Naive Bayes;
- Entscheidungsbäume, wie Random Forest;
- logistische Regression wie multinominale logistische Regression;
- neuronales Netzwerk;
- Entscheidungsbäume; und
- Bayes-Netzwerk.

4. Verfahren nach mindestens einem der vorstehenden Ansprüche, ferner umfassend das Trainieren eines Lernalgorithmus gemäß dem Trainingsdatensatz, der die historischen Daten (25) umfasst.

5. Verfahren nach Anspruch 4, wobei das Trainieren (25) das Trainieren des Lernalgorithmus gemäß dem Trainingsdatensatz, der mindestens eines von historischen In-vivo-Trainingsdaten und historischen In-vitro-Trainingsdaten umfasst, umfasst.

6. Verfahren nach Anspruch 4 und 5, wobei das Trainieren (25) das Trainieren des Lernalgorithmus gemäß dem Trainingsdatensatz, der historische kontinuierliche Überwachungsdaten umfasst, umfasst.

7. Verfahren nach mindestens einem der Ansprüche 4 bis 6, wobei das Trainieren (25) das Trainieren des Lernalgorithmus gemäß dem Trainingsdatensatz, der Testdaten aus der folgenden Gruppe umfasst: Herstellungstestdaten, Patiententestdaten, personalisierte Patiententestdaten, Populationstestdaten, die mehrere Patientendatensätze umfassen, umfasst.

8. Verfahren nach mindestens einem der Ansprüche 4 bis 7, wobei das Trainieren (25) das Trainieren des Lernalgorithmus gemäß dem Trainingsdatensatz, der Trainingsdaten umfasst, die einen oder mehrere sensorbezogene Parameter aus der folgenden Gruppe angeben: Stromwerte des Sensors (7), insbesondere im Falle einer kontinuierlichen Überwachung, Sensorstromwerte einer Arbeitselektrode; Spannungswerte des Sensors (7) oder Spannungswerte zwischen der Referenzelektrode und der Arbeitselektrode; Temperatur eines Umfeldes des Sensors (7) während der Messung; Empfindlichkeit des Sensors (7); Abweichung des Sensors (7); und Kalibrierungsstatus des Sensors (7), umfasst.

9. Verfahren nach Anspruch 8, wobei der eine oder die mehreren sensorbezogene(n) Parameter mindestens einen der folgenden einschließt/einschließen

- nichtkorrelierte sensorbezogene Parameter; und
- korrelierte sensorbezogene Parameter.

10. Verfahren nach mindestens einem der vorstehenden Ansprüche, sofern sie sich auf Anspruch 2 beziehen, ferner umfassend das Validieren des trainierten Lernalgorithmus gemäß einem Validierungsdatensatz (26), der gemessene kontinuierliche Überwachungsdaten und/oder simulierte kontinuierliche Überwachungsdaten umfasst, die mindestens eines von Folgendem für den Sensor (7) angeben: Herstellungsfehlerstatus, Fehlfunktionsstatus und Anamneseanzeigestatus.

11. Verfahren nach mindestens einem der vorstehenden Ansprüche, ferner umfassend

- das Validieren des trainierten Lernalgorithmus gemäß dem Validierungsdatensatz, der komprimierte Validierungsdaten umfasst;

wobei die komprimierten Validierungsdaten mittels mindestens einem von einem linearen Regressionsverfahren und einem Glättungsverfahren bestimmt werden.

12. Zustandsmaschinensystem (1) mit einem oder mehreren Prozessor(en) (2), das zum Datenverarbeiten und zum Durchführen eines Verfahrens zum Detektieren eines Betriebsstatus für einen Sensor (7) zur kontinuierlichen Glukosemessung ausgebildet ist, wobei das Verfahren Folgendes umfasst:

- Empfangen kontinuierlicher Überwachungsdaten (20), die sich auf einen Betrieb eines Sensors beziehen und komprimierte Überwachungsdaten umfassen;
- Bereitstellen eines trainierten Lernalgorithmus (21) zum Detektieren eines Betriebsstatus für den Sensor (7), der eine Sensorfunktion angibt, wobei der Lernalgorithmus gemäß einem Trainingsdatensatz trainiert wird, der historische Daten und komprimierte Trainingsdaten umfasst;
- Detektieren eines Betriebsstatus für den Sensor (7) durch Analysieren der kontinuierlichen Überwachungsdaten mit dem trainierten Lernalgorithmus (22); und
- Bereitstellen von Ausgabedaten (23), die den nachgewiesenen Betriebsstatus für den Sensor (7) angeben;

wobei

- die historischen Daten aus Daten bestehen, die mindestens eines von vor dem Detektieren des Betriebsstatus gesammelt, nachgewiesen und gemessen sind;

**dadurch gekennzeichnet, dass**:

- die komprimierten Überwachungsdaten und die komprimierten Trainingsdaten durch mindestens eines von einem linearen Regressionsverfahren und einem Glättungsverfahren bestimmt werden und das Ergebnis einer Reduktion der Dimension der Überwachungsdaten bzw. Trainingsdaten sind, wobei in den verschiedenen Stufen der Komprimierung die Überwachungsdaten bzw. Trainingsdaten Daten pro Sekunde, Daten pro Minute und/oder statistische Daten, die charakteristische Werte wie Sensorparameter, Varianz, Rauschen oder Änderungsrate einschließen, umfassen.

**Revendications**

1. Procédé mis en oeuvre par ordinateur pour détecter un état de fonctionnement d'un capteur de mesure de glucose en continu (7), le procédé, dans une machine d'état, comprenant

   - la réception de données de surveillance en continu (20) liées à un fonctionnement d'un capteur et comprenant des données de surveillance compressées ;
   - la fourniture d'un algorithme d'apprentissage entraîné (21) pour détecter un état de fonctionnement du capteur (7) qui signifie une fonction de capteur, dans lequel l'algorithme d'apprentissage est entraîné selon un ensemble de données d'entraînement comprenant des données d'historique et des données d'entraînement compressées ;
   - la détection d'un état de fonctionnement du capteur (7) par analyse des données de surveillance en continu avec l'algorithme d'apprentissage entraîné (22) ; et
   - la fourniture de données de sortie (23) indiquant l'état de fonctionnement détecté du capteur (7) ;

   dans lequel

   - les données d'historique sont constituées de données qui sont au moins un parmi collectées, détectées et mesurées avant la détection de l'état de fonctionnement ; **caractérisé en ce que** :
   - les données de surveillance compressées et les données d'entraînement compressées sont déterminées par au moins une parmi une méthode de régression linéaire et une méthode de lissage et sont le résultat de la réduction de la dimension des données de surveillance et des données d'entraînement, respectivement, dans lequel dans les différents stades de compression, les données de surveillance et les données d'entraînement, respectivement, comprennent des données par seconde, des données par minute et/ou des données statistiques incluant des valeurs caractéristiques telles que les paramètres de capteur, la variance, le bruit ou le taux de variation.

2. Procédé selon la revendication 1, dans lequel la détection comprend au moins une parmi

   - la détection d'un état de défaut de fabrication du capteur (7) indiquant un défaut dans un processus de fabrication du capteur (7) ;
   - la détection d'un état de dysfonctionnement (7) du capteur indiquant un dysfonctionnement du capteur (7) ;
   - la détection d'un état d'anomalie du capteur indiquant une anomalie dans le fonctionnement du capteur (7) ; et - la détection d'un état indiquant une anamnèse du capteur (7) indiquant un état de patient anamnestique pour le patient pour lequel les données de surveillance en continu sont fournies.

3. Procédé selon la revendication 1 ou 2, dans lequel la fourniture de l'algorithme d'apprentissage entraîné (21) comprend la fourniture d'au moins un algorithme d'apprentissage choisi dans le groupe suivant

   - K-ième voisin le plus proche ;
   - machines à vecteurs de support ;
   - Bayes naïf ;
   - arbres de décision tels qu'une forêt aléatoire ;
   - régression logistique telle qu'une régression logistique multinomiale ;
   - réseau neuronal ;
   - arbres de décision ; et
   - réseau bayésien.

4. Procédé selon au moins une des revendications précédentes, comprenant en outre l'entraînement d'un algorithme d'apprentissage selon l'ensemble de données d'entraînement comprenant les données d'historique (25).

5. Procédé selon la revendication 4, dans lequel l'entraînement (25) comprend l'entraînement de l'algorithme d'entraînement selon l'ensemble de données d'entraînement comprenant au moins un parmi les données d'entraînement d'historique *in vivo* et les données d'entraînement d'historique *in vitro*.

6. Procédé selon la revendication 4 et 5, dans lequel l'entraînement (25) comprend l'entraînement de l'algorithme d'apprentissage selon l'ensemble de données d'entraînement comprenant les données d'historique de surveillance en continu.

**7.** Procédé selon au moins l'une des revendications 4 à 6, dans lequel l'entraînement (25) comprend l'entraînement de l'algorithme d'entraînement selon l'ensemble de données d'entraînement comprenant des données de test issues du groupe suivant : les données de test de fabrication, les données de test de patient, les données de test de patient personnalisées, les données de test de population comprenant plusieurs ensembles de données de patients.

**8.** Procédé selon au moins l'une des revendications 4 à 7, dans lequel l'entraînement (25) comprend l'entraînement de l'algorithme d'apprentissage selon l'ensemble de données d'apprentissage comprenant les données d'entraînement indiquant un ou plusieurs paramètres liés au capteur issus du groupe suivant : les valeurs actuelles du capteur (7), en particulier dans le cas de valeurs actuelles de capteur de surveillance en continu d'une électrode de travail ; les valeurs de tension du capteur (7) ou les valeurs de tension entre l'électrode de référence et l'électrode de travail ; la température d'un environnement du capteur (7) pendant la mesure ; la sensibilité du capteur (7) ; le décalage du capteur (7) ; et l'état d'étalonnage du capteur (7).

**9.** Procédé selon la revendication 8, dans lequel le ou les paramètres liés au capteur incluent au moins un parmi

- des paramètres liés au capteur non corrélés ; et
- des paramètres liés au capteur corrélés.

**10.** Procédé selon au moins l'une des revendications précédentes, dans la mesure où il se réfère à la revendication 2, comprenant en outre la validation de l'algorithme d'apprentissage entraîné selon un ensemble de données de validation (26) comprenant les données de surveillance en continu mesurées et/ou les données de surveillance en continu simulées indiquant, du capteur (7), au moins un parmi : un état de défaut de fabrication, un état de dysfonctionnement et un état indiquant une anamnèse.

**11.** Procédé selon au moins l'une des revendications précédentes, comprenant en outre

- la validation de l'algorithme d'apprentissage entraîné selon l'ensemble de données de validation comprenant les données de validation compressées ;

dans lequel les données de validation compressées sont déterminées par au moins une parmi une méthode de régression linéaire et une méthode de lissage.

**12.** Système de machine d'état (1), ayant un ou plusieurs processeurs (2) configurés pour traiter les données et pour réaliser un procédé de détection d'un état de fonctionnement d'un capteur de mesure de glucose en continu (7), le procédé comprenant

- la réception de données de surveillance en continu (20) liées à un fonctionnement d'un capteur et comprenant des données de surveillance compressées ;
- la fourniture d'un algorithme d'apprentissage entraîné (21) pour détecter un état de fonctionnement du capteur (7) qui signifie une fonction de capteur, dans lequel l'algorithme d'apprentissage est entraîné selon un ensemble de données d'entraînement comprenant des données d'historique et des données d'entraînement compressées ;
- la détection d'un état de fonctionnement du capteur (7) par analyse des données de surveillance en continu avec l'algorithme d'apprentissage entraîné (22) ; et
- la fourniture de données de sortie (23) indiquant l'état de fonctionnement détecté du capteur (7) ;

dans lequel

- les données d'historique sont constituées de données qui sont au moins un parmi collectées, détectées et mesurées avant la détection de l'état de fonctionnement ; **caractérisé en ce que** :
- les données de surveillance compressées et les données d'entraînement compressées sont déterminées par au moins une parmi une méthode de régression linéaire et une méthode de lissage et sont le résultat de la réduction de la dimension des données de surveillance et des données d'entraînement, respectivement, dans lequel dans les différents stades de compression, les données de surveillance et les données d'entraînement, respectivement, comprennent des données par seconde, des données par minute et/ou des données statistiques incluant des valeurs caractéristiques telles que les paramètres de capteur, la variance, le bruit ou le taux de variation.

Fig. 1

Fig. 2

Fig. 3

Fig. 4

Fig. 5

Fig. 6

Fig. 7

Fig. 8

Binary + few parameters
*Error ~190 +A2+D*

ACC: 95%

All result classes + parameters
*Error ~190 +A2+D+S+St40+...+*

ACC: 90%

Optimization
*k Fold Cross Validation*

ACC: 93%

Result Model

Fig. 9

Fig. 10

Fig. 11

Fig. 12

Fig. 13

33

Fig. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140182350 A1
- EP 2335584 A2
- US 2015164386 A1
- US 2010323431 A1

**Non-patent literature cited in the description**

- **SHMUELI et al.** Data Mining for Business analytics - Concepts, Techniques, and Applications with XLMiner. John Wiley & Sons, 2016
- **DOMINGOS.** A Few Useful Things to Know About MachineLearning. *Commun. ACM,* 2012, vol. 55 (10), 78-87
- **SAMMUT et al.** Encyclopedia of Machine Learning. Springer Publishing Company, 2011
- **BERTHOLD et al.** Guide to Intelligent Data Analysis: How to Intelligently Make Sense of Real Data. Springer Publishing Company, 2010
- **LANDIS et al.** The Measurement of Observer Agreement for Categorical Data,. *Biometrics,* 1977, vol. 33, 159-174
- **BACKHAUS et al.** Multivariate Analysemethoden: Eine anwendungsorientierte Einführung. Springer, 2015
- An Introduction to Generalized Linear Models. **DOBSON.** Chapman & Hall/CRC Texts in Statistical Science. Taylor & Francis, 2010
- **SHEATHER.** A Modern Approach to Regression with R. Springer Science & Business Media, 2009
- **AHO KET.** Model selection for ecologists: the worldviews of AIC and BIC. *Ecology,* 2014, vol. 95, 631-636
- **BREIMAN.** Random Forests. *Mach. Learn,* 2001, vol. 45 (1), 5-32